(19) Europäisches Patentamt
European Patent Office
Office européen des brevets

(11) Numéro de publication: **0 323 290 B1**

(12) # FASCICULE DE BREVET EUROPEEN

(45) Date de publication de fascicule du brevet: **11.11.92**

(51) Int. Cl.5: **C07C 45/36**, C07C 47/565

(21) Numéro de dépôt: **88403071.9**

(22) Date de dépôt: **05.12.88**

(54) **Procédé de production d'aldéhydes aromatiques.**

(30) Priorité: **24.12.87 FR 8718133**

(43) Date de publication de la demande:
**05.07.89 Bulletin 89/27**

(45) Mention de la délivrance du brevet:
**11.11.92 Bulletin 92/46**

(84) Etats contractants désignés:
**AT BE CH DE ES FR GB GR IT LI LU NL SE**

(56) Documents cités:
**EP-A- 0 012 939**
**US-A- 3 666 815**
**US-A- 4 471 140**

**CHEMICAL ABSTRACTS, vol. 90, 1979, page 629, résumé no. 186588j, Columbus, Ohio, US; CS-A-176 495**

(73) Titulaire: **L'AIR LIOUIDE, SOCIETE ANONYME POUR L'ETUDE ET L'EXPLOITATION DES PROCEDES GEORGES CLAUDE**
**75, Ouai d'Orsay**
**F-75321 Paris Cédex 07(FR)**

(72) Inventeur: **Campo, Philippe**
**13, rue de Carnac**
**F-78180 Montigny-le-Bretonneux(FR)**
Inventeur: **Cocolios, Panayotis**
**44, rue de Marcoussis**
**F-91470 Limours(FR)**
Inventeur: **Dognin, Paul**
**62, rue Brancion**
**F-75015 Paris(FR)**
Inventeur: **Ledon, Henry**
**1 bis, avenue de l'Assemblée Nationale**
**F-78000 Versailles(FR)**

(74) Mandataire: **Vesin, Jacques et al**
**L'AIR LIOUIDE, SOCIETE ANONYME POUR L'ETUDE ET L'EXPLOITATION DES PROCEDES GEORGES CLAUDE 75, quai d'Orsay**
**F-75321 Paris Cédex 07(FR)**



Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne un procédé de production d'aldéhydes aromatiques, en particulier de p-hydroxybenzaldéhydes.

Les p-hydroxybenzaldéhydes sont des molécules bifonctionnelles participant à une grande variété de réactions caractéristiques des groupes phénol et aldéhyde. Ces aldéhydes aromatiques, notamment le p-hydroxybenzaldéhyde sont des intermédiaires dans la synthèse de divers produits via des réactions d'hydrogénation catalytique, d'oxydation, de Canizzaro, réactions avec des composés possèdant un méthylène actif, synthèses hétérocycliques, réactions avec des amines ou des amides, formation de cyanophénol ou d'éther, halogénation, nitration, formation de phénylglycine ou de sels.

Les applications les plus courantes du p-hydroxybenzaldéhyde se recontrent en agrochimie, industrie alimentaire, pharmacie, industries des polymères et fibres synthétiques et en galvanoplastie.

Industriellement, selon le procédé dit Reimer-Teimann, brevet US. 3.365.500, on obtient le p-hydroxybenzaldéhydes, à partir du phénol mis en réaction avec le chloroforme et l'hydroxyde de sodium. Ce procédé conduit à un mélange d'isomères ortho/para,avec un rendement de 60 à 80 % par rapport au phénol. Bien que les deux produits soient indépendamment valorisables, après séparation par distillation, le prix de revient est élevé. De plus, la formation de sels de sodium mélangés avec le phénol n'ayant pas réagi, exige un traitement de récupération du produit de départ économiquement peu rentable, et un traitement pour rendre l'effluent écologiquement acceptable.

Selon le brevet US. 4.119.671, on a proposé la condensation du formaldéhyde sur le formol en milieu basique qui conduit principalement à un mélange d'hydroxyméthyl-2 et 4-phénols, ensuite catalytiquement oxydés à l'oxygène en aldéhydes. Toutefois, ce procédé s'avère difficile à mettre en oeuvre à cause de la grande réactivité du système formaldéhyde/phénol qui provoque la formation de mélanges très complexes. En outre, l'exploitation de ce procédé est limité par la difficulté de séparation des deux hydroxyméthylphénols du mélange en présence de base. Car cette opération qui obère le rendement global du procédé est néanmoins indispensable avant l'étape d'oxydation.

L'oxydation des p-hydroxytoluènes en p-hydroxyaryldéhydes par l'oxygène moléculaire a également été envisagés. Ces réactions d'oxydation sont catalysées par des sels organiques et inorganiques de cobalt, manganèse, chrome ou nickel en présence de soude dans le méthanol. Les rendements varient de 40 à 80 % et dépendent à la fois des substrats mis en oeuvre et des conditions opératoires. Suivant ce type de procédé, la demande de brevet européen 0012939, préconise l'obtention de dérivés du 4-hydroxybenzaldéhyde en présence d'une quantité catalytique d'un composé du cobalt ou du cobalt métallique; en indiquant des taux de conversion et de sélectivité assez élevés, mais d'une manière globale sans isolement du produit recherché.

Pour rendre l'obtention de p-hydroxybenzaldéhydes économiquement intéressante et écologiquement acceptable, il convient de rechercer un procédé utilisant un produit de départ bon marché, comprenant un nombre minimum d'étapes réactionnelles, dans la mesure du possible sans séparations intermédiaires, opérant dans des conditions douces, conduisant à un seul isomère avec une conversion élevée et libérant des effluents peu chargés en matière organique et sels minéraux.

L'oxydation d'alkylphénols, en particulier du méthyl-4 phénol ou d'un dérivé substitué, représentés par la formule

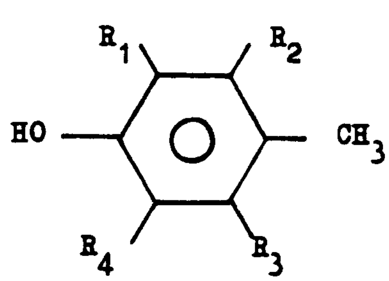

dans laquelle $R_1$, $R_2$, $R_3$ et $R_4$ sont ensemble ou séparément l'hydrogène, un halogène ou un radical alcoyle inférieur contenant 1 à 4 atomes de carbone, en milieu solvant, en présence d'une base, et catalysée par des sels organiques ou inorganiques des métaux de transition, semble la mieux adaptée à l'obtention industrielle et spécifique des aldéhydes aromatiques représentés par la formule

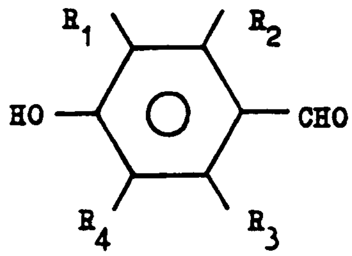

dans laquelle $R_1$, $R_2$, $R_3$ et $R_4$ ont les mêmes significations que précédemment, à condition, par un choix judicieux du catalyseur, d'améliorer à la fois la conversion du substrat et la sélectivité en aldéhyde sous forme de produit isolé, purifié, avec possibilité de recyclage du catalyseur.

La présente invention permet d'atteindre cet objectif, en conduisant la réaction d'oxydation en présence d'un complexe du cobalt (II) du type complexe chélaté de cobalt de structure rigide peu oxydable. Ce complexe est de préférence le bis-(4-méthylpyridine -isoindolinato) cobalt (II) acétate (A), le phtalocyaninato-cobalt (II) (B) ou le sulfophtalocyaninato-cobalt (II) (C).

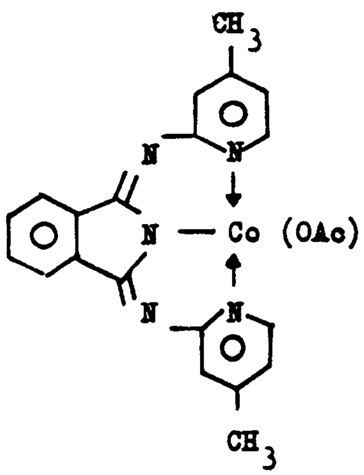

(A)

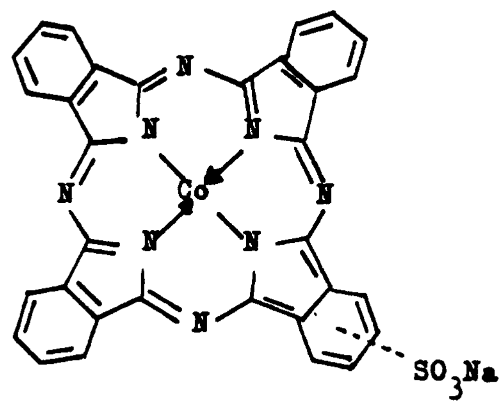

(B) (C)

Le complexe chélaté est mis en oeuvre en quantité catalytique, comprise entre 1‰, et 5% par rapport au substrat alkylphénol, en particulier entre 0,5% et 2 %.

La réaction d'oxydation peut être conduite en phase homogène en présence de l'un ou l'autre des complexes proposés, ou en suspension après adsorption du complexe (B) ou (C) sur un support tel que le charbon actif.

Dans des conditions réactionnelles identiques le bis-(4-méthylpyridine-isoindolinato-cobalt (II) acétate (A) et le sulfophtalocyaninato-cobalt (II) (C) ont des performances voisines. Il en est de même du catalyseur (B) ou (C) déposé sur charbon. De plus, dans ce dernier cas, il a été observé un déroulement plus aisé de la phase d'extraction et l'obtention d'un produit brut plus propre. En outre, on peut envisager la réutilisation du catalyseur, après lavage, successivement à l'eau et par un alcool, puis séchage, par exemple à l'étuve vers 80° C, sans perte de ses performances.

L'oxydation du méthyl-4 phénol (p-crésol) à l'oxygène catalysée par le complexe (A), (B) ou (C) en milieu méthanolique basique conduit exclusivement au p-hydroxybenzaldéhyde. Dans des conditions opératoires douces (température 60° C, pression d'oxygène 0,1 MPa, solvant méthanol), la conversion du substrat atteint 100 % entre 10 et 15 heures, avec une sélectivité et un rendement et aldéhyde supérieur à 90 %, calculée d'après la masse du produit isolé.

L'oxydation des alkylphénols est conduite en présence d'oxygène, sous forme d'oxygène pur ou de mélanges oxygène-azote, en proportions adaptées, à teneur élevée en oxygène, comprise entre 40 et 90 % en volume, par exemple 80 % en volume d'oxygène - 20 % en volume d'azote. En présence d'air il a été constaté que la cinétique réactionnelle est moins favorable.

La conduite de l'oxydation par l'oxygène sous la pression normale (environ 0,1 MPa) donne d'excellents

résultats.

Le solvant joue un rôle important dans l'évolution de la réaction. Les alcools constituent de bons solvants et les meilleurs résultats sont obtenus avec le méthanol. Ceux-ci s'améliorent en fonction de la diminution de la teneur en eau du méthanol. Le méthanol anhydre convient parfaitement, et l'on atteint des rendements supérieurs à 90 % en p-hydroxybenzaldéhyde isolé, sous une pression de 0,1 MPa à 60 ° C, alors qu'avec des mélanges méthanol-eau les rendements sont très nettement inférieurs, et dans l'eau pure le rendement est extrèmement limité.

En milieu solvant méthanol, on observe une évolution de la réaction d'oxydation dès 20 ° C, avec une amélioration des résultats à 30 ° C, et une optimisation aux environ de 60 ° C. La conduite de l'oxydation entre 55 et 65 ° C peut être avantageusement envisagée.

Le solvant est utilisé en quantité minimale, pour la solubilisation du substrat à oxyder, de manière à obtenir la concentration maximale en p-crésol de départ permettant d'obtenir un milieu liquide homogène.

L'oxydation peut être conduite en présence de divers types de bases, tels que les hydroxydes alcalins et les alcoxydes alcalins, cependant les meilleurs résultats sont obtenus avec l'hydroxyde de sodium, de préférence mis en oeuvre dans un rapport Na OH/substrat compris entre 1 à 6, notamment entre 2,5 et 4,5, en particulier environ 3,5. Il existe un rapport base/substrat optimum et une concentration optimale du para-crésol dans le milieu solvant méthanol pour atteindre une conversion de 100 % et une sélectivité en aldéhyde supérieure à 90 %.

L'évolution de la réaction d'oxydation est suivie par chromatographie liquide haute performance (CLHP) et par la consommation d'oxygène en fonction du temps.

Après acidification du mélange réactionnel, le para-hydroxy-benzaldéhyde est extrait à l'acétate d'éthyle, lavé à l'eau et séché sous pression réduite et recristallisé dans un mélange de solvants dichlorométhane/n-hexane dans un rapport 1/1. Le produit isolé après recristallisation est identifié par comparaison de ses spectres de résonance magnétique nucléaire RMN$^1$H et infra-rouge IR avec ceux d'un échantillon commercial de p-hydroxybenzaldéhyde, par son point de fusion (117 ° C) et par le point de fusion du dérivé de condensation avec la p-nitrophénylhydrazine (266 ° C).

Le procédé de l'invention est applicable à l'obtention industrielle d'aldéhydes aromatiques, à partir d'alkylphénols, en particulier du méthyl-4 phénol, (p-crésol), du diméthyl-2,4-phénol, du triméthyl-2,4,6 phénol, du ditertiobutyl-2,6-méthyl-4 phénol, et du bromo-2-méthyl-4-phénol.

L'effet du substituant sur la consommation d'oxygène en fonction du temps d'oxydation a été étudié, notamment pour un substituant halogéné, le brome, un mono substituant alcoyle, le méthyl-2, et des substituants alcoyles, le diméthyl-2,6 et le ditertiobutyl-2,6. Il a été observé que les alkylphénols porteurs de substituants donneurs d'électrons tels les substituants alcoyles, s'oxydent plus rapidement que le méthyl-4 phénol. Par contre les alkylphénols porteurs de substituants attracteurs d'électrons, tel le bromo-2 crésol s'oxydent plus lentement que le para-crésol.

Comme précédemment, après arrêt de la réaction d'oxydation, on acidifie le milieu réactionnel, on isole l'hydroxy aldéhyde aromatique, lave et purifie le produit par recristallisation dans des solvants adaptés au produit.

Il est donné ci-après à titre non limitatif des exemples de synthèse d'aldéhydes aromatiques.

Exemple 1

Oxydation catalytique du p-crésol (méthyl-4 phénol) par l'oxygène

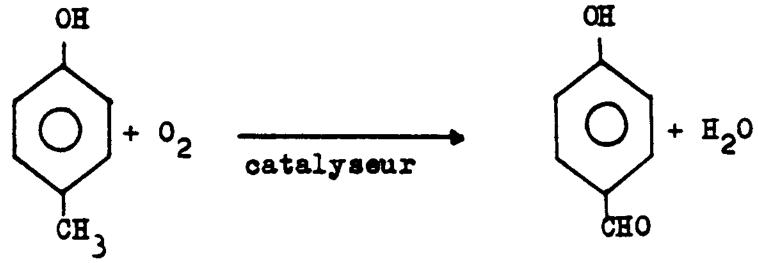


Dans les différents essais, l'oxydation a été conduite dans les conditions suivantes :
méthyl-4 phénol : 50 mmol, solvant le méthanol MeOH; hydroxyde alcalin : NaOH, pression d'oxygène $PO_2$ = 0,1 MPa, température 60 ° C, le catalyseur est mis en oeuvre à raison de 0,5 mmol sous forme de bis-(4-méthylpyridine-isoindolinato) cobalt (II) acétate (A) (4-MeBPI) Co(OAc), de sulfophtalocyaninato-cobalt (II)

PcSCo (C), sur charbon (C) PcSCo/Ch, de (C) PcSCo/Ch recyclé désigné par (R) et de phtalocyaninato-cobalt (II) (B) sur charbon PcCo/Ch.

Les résultats obtenus sont consignés dans le Tableau I ci-après, dans lequel le p-crésol constitue le substrat désigné par : sub. La sélectivité : Sel = [aldéhyde]/[Substrat consommé]x100. Le rendement : Rdt = [Aldéhyde]/[substrat initial] x 100.

$[O_2]/[Ald]$ = nombre de moles d'oxygène consommé par mole d'aldéhyde produit isolé.

(1) Bis (2-méthoxyéthyl) éther = diglyme

(2) : Isolement par précipitation dans l'eau

(3) : 10 % de (p-hydroxybenzyl) méthyléther

(*) : Conversion du substrat = [substrat consommé] / [substrat initial] x 100.

## TABLEAU I

| Essai | Conditions | | ALDEHYDE Isolé | SUBSTRAT Consommé | $O_2$ Consommé | $[O_2]/[Ald]$ |
|---|---|---|---|---|---|---|
| 1 | (4-MeBPI)Co(OAc) | mmol | 15.6 | 35.4 | 59.3 | 3.8 |
| | MeOH 75ml | Sel | 44.1 | | | |
| | NaOH/Sub = 2.5 | Rdt | 31.1 | 70.8* | | |
| 2 | (4-MeBPI)Co(OAc) | mmol | 14.4 | 40.1 | 57.4 | 3.9 |
| | MeOH 150ml | Sel | 35.9 | | | |
| | NaOH/Sub = 2.5 | Rdt | 28.8 | 80.2* | | |
| 3 | (4-MeBPI)Co(OAc) | mmol | 28.7 | 49.1 | 103 | 3.6 |
| | MeOH 32ml | Sel | 58.5 | | | |
| | NaOH/Sub 2.5 | Rdt | 57.4 | 98.2* | | |
| 4 | (4-MeBPI)Co(OAc) | mmol | 7.4 | 31.5 | 44.7 | 6 |
| | MeOH 32ml | Sel | 23.6 | | | |
| | NaOH/Sub = 1.5 | Rdt | 14.9 | 63.0* | | |
| 5 | (4-MeBPI)Co(OAc) | mmol | 25.8 | 50 | 103 | 4 |
| | Pipéridine | Sel | 51.7 | | | |
| | MeOH 32 ml | Rdt | 51.7 | 100* | | |
| | NaOH/Sub = 2.5 | | | | | |
| 6 | (4-MeBPI)Co(OAc) | mmol | 39.9 | 50 | 67 | 1.7 |
| | MeOH 32ml | Sel | 79.9 | | | |
| | NaOH/Sub = 3.5 | Rdt | 79.9 | 100* | | |

TABLEAU I (Suite)

| Essai | Conditions | | ALDEHYDE Isolé | SUBSTRAT Consommé | $O_2$ Consommé | $[O_2]/[Ald]$ |
|---|---|---|---|---|---|---|
| 7 | PcSCo | mmol | 41.4 | 50 | 80.7 | 1.9 |
| | MeOH 32 ml | Sel | 82.7 | | | |
| | NaOH/Sub = 3.5 | Rdt | 82.7 | 100* | | |
| 8 | PcSCo/Charbon | mmol | 42.7 | 50 | 71.1 | 1.7 |
| | MeOH 32ml | Sel | 85.5 | | | |
| | NaOH/Sub = 3.5 | Rdt | 85.8 | 100* | | |
| 9 | PcSCo/Ch (R) | mmol | 44.1 | 50 | 66.6 | 1.5 |
| | MeOH 32 ml | Sel | 88.2 | | | |
| | NaOH/Sub = 3.5 | Rdt | 88.2 | 100* | | |
| 10 | PcSCo/Ch | mmol | (3)46 | 50 | 72.2 | 1.6 |
| | MeOH 32 ml | Sel | 92 | | | |
| | NaOH/Sub = 3.5 | Rdt | 92 | 100* | | |
| | Diglyme(1)0.6ml | | | | | |
| 11 | PcCo/Ch (2) | mmol | 31.5 | 34 | 91 | 2.9 |
| | MeOH 32ml | Sel | 93.5 | | | |
| | NaOH/Sub = 3.5 | Rdt | 63 | 67* | | |

Exemple 2

Synthèse d'hydroxybenzaldéhydes substitués.

2-1. Réaction

Dans un dicol de 250 ml équipé d'un réfrigérant à eau et d'un septum, on introduit, sous atmosphère d'oxygène, 175 mmole (7g) de soude en pastilles, 0,5 mmole de sulfophtalocyananine de cobalt adsorbée sur 3,75g de charbon activé (120 à 200 mesh), 50 mmole d'alkylphénol, (25 mmole dans le cas du di-tert-butyl-3,6-méthyl-4-phénol),et 30 ml de méthanol anhydre (99,95 %). Le mélange est porté à 60 °C par immersion dans un bain d'huile et maintenu sous agitation. L'oxygène est admis dans le réacteur par le haut de réfrigérant sous 0,105 MPa absolus. Une soupape de mercure réglée à 0,108 MPa absolus assure l'étanchéité du montage.

L'avancement de la réaction est suivi par :

- analyse CLHP d'échantillons de mélange réactionnel prélevés périodiquement à l'aide d'une seringue ;
- enregistrement de la courbe de consommation d'oxygène en fonction du temps, $O_2 = f(t)$.

2-2. Dosage et isolement

Après arrêt de la réaction, le catalyseur est isolé par filtration sous pression réduite (trompe à eau) et lavé successivement au méthanol, à l'eau, puis au méthanol pour entraîner le maximum de produits organiques. Le volume du filtrat est ajusté à 500ml par addition de méthanol. Un prélèvement de 5ml de cette solution sert au dosage par CLHP du substrat restant et de l'aldéhyde formé, après addition de l'étalon adéquat. La solution principale est acidifiée par de l'acide chlorhydrique 3,3 N jusqu'à pH 4,7.

La phase de l'isolement dépend du produit mis en oeuvre :
a) di-tert-butyl-3,5-hydroxy-4-benzaldéhyde :

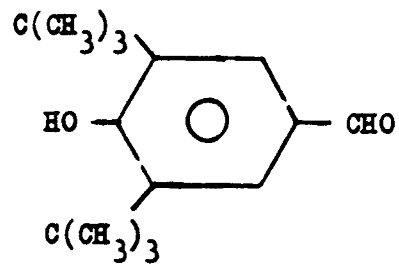

Après acidification on observe la formation d'un précipité qui est filtré sous pression réduite, lavé à l'eau, puis séché sur $P_2O_5$ (19 Pa) P.F. = 185° C, m = 2,45g (10,5 mmole), Rdt = 42 %.
b) hydroxy-4-méthyl-3-benzaldéhyde :

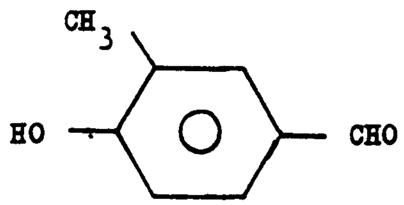

Après acidification, le filtrat est évaporé à sec. Le résidu solide est repris par l'acétonitrile. Les sels minéraux présents sont insolubles et éliminés par filtration. Le filtrat après évaporation, conduit à une huile contenant 70 % d'aldéhyde et 30 % de produit de départ (contrôle RMN H1 et CLHP). L'aldéhyde est isolé par traitements successifs de l'huile aux mélanges $CH_2Cl_2$/n-$C_6H_{14}$ 1/10 et $Et_2O$/n-$C_6H_{14}$ 1/10 et séché sur $P_2O_5$ (19 Pascals)
P.F. = 109° C, m = 2,40 g (17,6 mmole), Rdt = 35 %.
c) hydroxy-4-diméthyl-3,5-benzaldéhyde :

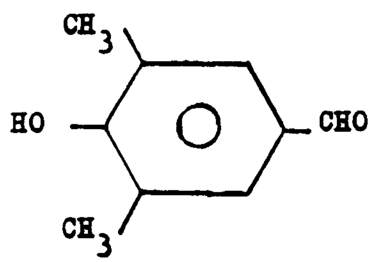

Après acidification et réduction du volume des solvants par évaporation sous pression réduite on obtient un précipité. Après filtration et lavage à l'eau on le sèche sur $P_2O_5$ (19 Pa).
P.F. = 116° C, m = 4,1 g (27,3 mmole), Rdt = 55%.
d) bromo-3-hydroxy-4-benzaldéhyde :

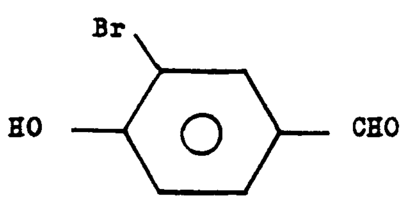

L'aldéhyde isolé comme dans le cas précédent contient 10 % de produit de départ (contrôle RMN H1)

qui est aisément éliminé par lavage du résidu solide à l'hexane.
P.F. = 132 °C, m = 5,28g (26,3 mmole), Rdt = 53 %.

2-3. Analyse chromatographique CLHP

colonne WATERS NOVAPACK C18
détection UV : 270nm
éluant : A/B/C = MeOH/MeCN/$H_2O$ pH = 4 ($H_2SO_4$)

| ALDEHYDE | ELUANT A/B/C | DEBIT ml/min | ETALON |
|---|---|---|---|
| DITERTBUTYL | 40/40/20 | 0,5 | méthyl-3-benzonitrile |
| METHYL | 20/20/60 | 1,0 | benzonitrile |
| DIMETHYL | 20/20/60 | 0,5 | hydroxy-4-benzonitrile |
| BROMO | 20/20/60 | 1,0 | hydroxy-4-benzonitrile |

Les résultats obtenus sont consignés dans le tableau II ci-après

**TABLEAU II**

| ESSAI | SUBSTRAT SUBSTITUANT | | ALDEHYDE CLHP | ALDEHYDE Isolé | SUBSTRAT consommé | $O_2$ consommé | [$O_2$]/[Ald] CLHP |
|---|---|---|---|---|---|---|---|
| 1 | DITERT BUTYL-2,6 | mmol | 11.7 | 10.5 | 25* | 65 | 5.6 |
| | | Sel | 47 | 42 | | | |
| | | Rdt | 47 | 42 | (100) | | |
| 2 | METHYL-2 | mmol | 21.9 | 17.6 | 45 | 74 | 3.4 |
| | | Sel | 49 | 39 | | | |
| | | Rdt | 44 | 35 | (89) | | |
| 3 | DIMETHYL-2,6 | mmol | 33 | 27.3 | 50 | 103 | 3.1 |
| | | Sel | 66 | 55 | | | |
| | | Rdt | 66 | 55 | (100) | | |

TABLEAU II (Suite)

| ESSAI | SUBSTRAT SUBSTITUANT | | ALDEHYDE CLHP | Isolé | SUBSTRAT consommé | $O_2$ consommé | $[O_2]/[Ald]$ CLHP |
|---|---|---|---|---|---|---|---|
| 4 | BROMO-2 | mmol | 32.9 | 27.4 | 34 | 50 | 1.5 |
| | | Sel | 97 | 81 | | | |
| | | Rdt | 68 | 55 | (70) | | |

( ) : conversion du substrat

* : Substrat utilisé = 25 mmol

Température : 60°C

Substrat : 50 mmol

Soude : 175 mmol

Catalyseur : 0,5 mmol

Solvant : méthanol 30 ml.

## Revendications

1. Procédé de production d'aldéhydes aromatiques représentés par la formule

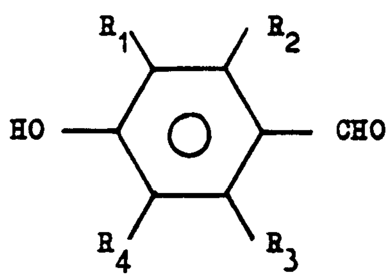

dans laquelle $R_1$, $R_2$, $R_3$ et $R_4$ sont ensemble ou séparément l'hydrogène, un radical alcoyle inférieur ou un halogène, par réaction d'un alkylphénol de formule

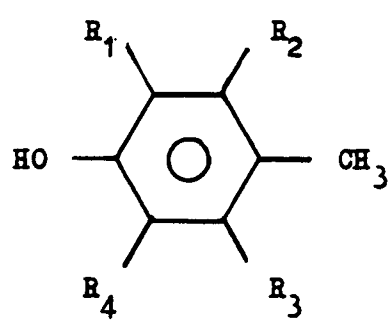

dans laquelle $R_1$, $R_2$, $R_3$, $R_4$ ont les mêmes significations que précédemment, avec de l'oxygène, en milieu solvant en présence d'une base et d'une quantité catalytique d'un composé du cobalt, caractérisé en ce que la réaction d'oxydation du substrat est conduite en présence d'un catalyseur constitué par un complexe chélaté de cobalt (II) de structure rigide peu oxydable choisi dans le groupe comprenant le bis-(4-méthylpyridine-isoindolinato) cobalt (II) acétate, le phtalocyaninato-cobalt (II) et le sulfophtalocyaninato-cobalt (II).

**2.** Procédé de production d'aldéhydes aromatiques selon la revendication 1, caractérisé en ce que le phtalocyananito-cobalt (II) et le sulfophtalocyaninato-cobalt (II) sont adsorbés sur support.

**3.** Procédé de production d'aldéhydes aromatiques selon la revendication 1, caractérisé en ce que la réaction d'oxydation est conduite en présence de 1‰ et 5% de catalyseur par rapport à l'alkylphénol.

**4.** Procédé de production d'aldéhydes aromatiques selon une quelconque des revendications 1 à 3, caractérisé en ce que le gaz oxydant est constitué par de l'oxygène pur ou des mélanges oxygène-azote à teneur élevée en oxygène, comprise entre 40 et 90 % en volume.

**5.** Procédé de production d'aldéhydes aromatiques selon une quelconque des revendications 1 à 4, caractérisé en ce que l'oxydation est conduite sous pression normale d'environ 0,1 MPa.

**6.** Procédé de production d'aldéhydes aromatiques selon une quelconque des revendications 1 à 5, caractérisé en ce que le milieu solvant est le méthanol anhydre utilisé en quantité minimale pour la solubilisation du substrat.

**7.** Procédé de production d'aldéhydes aromatiques selon une quelconque des revendications 1 à 6, caractérisé en ce que la réaction d'oxydation est mise en oeuvre à une température comprise entre 55 et 65 ° C, de préférence à 60 ° C.

**8.** Procédé de production d'aldéhydes aromatiques selon une quelconque des revendications 1 à 7, caractérisé en ce que la base constituée par l'hydroxyde de sodium, est mise en oeuvre dans un rapport NaOH/Substrat, compris entre 1 et 6, de préférence entre 2,5 et 4,5.

**9.** Procédé de production d'aldéhydes aromatiques selon une quelconque des revendications 1 à 8, caractérisé en ce que l'alkylphé nol est le méthyl-4 phénol, le diméthyl-2,4-phénol, le triméthyl-2,4,6 phénol, le ditertiobutyl-2,6 méthyl-4 phénol ou le bromo-2-méthyl-4 phénol.

**10.** Procédé de production d'aldéhydes aromatiques représentés par la formule

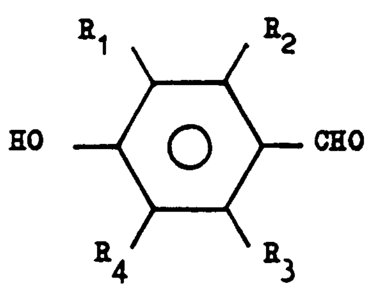

dans laquelle $R_1$, $R_2$, $R_3$, $R_4$ sont ensemble ou séparément l'hydrogène ou un radical alcoyle inférieur ou un halogène par réaction d'un alkylphénol de formule

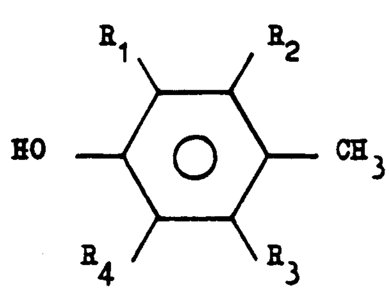

dans laquelle $R_1$, $R_2$, $R_3$ et $R_4$ ont les mêmes significations que précédemment, avec de l'oxygène, en milieu solvant, en présence d'une base et d'une quantité catalytique d'un composé du cobalt, caractérisé en ce que la réaction d'oxydation du substrat est conduite dans les conditions suivantes :

a) en présence d'un catalyseur constitué par un complexe chélaté de cobalt (II) de structure rigide

peu oxydable choisi dans le groupe comprenant le bis-(4-méthylpyridine-isoindolinato) cobalt (II) acétate, le phtalocyaninato-cobalt (II) et le sulfophtalocyaninato-cobalt (II) ;
b) le catalyseur est mis en oeuvre en quantité compris entre 1‰, et 5% par rapport au substrat ;
c) le gaz oxydant est constitué par de l'oxygène pur ou des mélanges oxygène-azote à teneur élevée en oxygène ;
d) l'oxydation est conduite sous pression normale et à température comprise entre 55 et 65 ° C ;
e) la base constituée par l'hydroxyde de sodium est mise en oeuvre dans un rapport NaOH/substrat compris entre 1 et 6.

## Claims

**1.** Process for the production of aromatic aldehydes represented by the formula

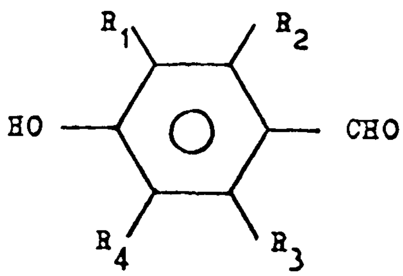

in which $R_1$, $R_2$, $R_3$ and $R_4$ are together or separately hydrogen, a lower alkyl radical or a halogen atom, by the reaction of an alkyl phenol of the formula

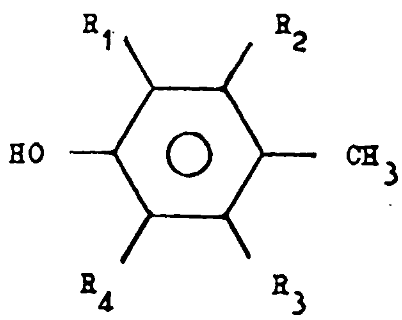

in which $R_1$, $R_2$, $R_3$, $R_4$ have the same meaning as before, with oxygen, in a solvent medium in the presence of a base and a catalytic quantity of a cobalt compound, characterised in that the substrate oxidation reaction is carried out in the presence of a catalyst constituted by a poorly oxidizable, chelate complex of cobalt (II) with a rigid structure chosen from the group comprising bis-(4-methylpyridine-isoindolinato) cobalt (II) acetate, phthalocyaninato-cobalt (II) and sulphophthalocyaninato-cobalt (II).

**2.** Process for the production of aromatic aldehydes according to claim 1, characterised in that phthalocyananito-cobalt (II) and sulphophthalocyaninato-cobalt (II) are adsorbed on a carrier.

**3.** Process for the production of aromatic aldehydes according to claim 1, characterised in that the oxidation reaction is carried out in the presence of 1‰ and 5% of catalyst relative to the alkyl phenol.

**4.** Process for the production of aromatic aldehydes according to any one of claims 1 to 3, characterised in that the oxidizing gas is constituted by pure oxygen or oxygen-nitrogen mixtures having a high oxygen content of between 40 and 90% by volume.

**5.** Process for the production of aromatic aldehydes according to any one of claims 1 to 4, characterised in that oxidation is carried out under a normal pressure of approximately 0.1 MPa.

6. Process for the production of aromatic aldehydes according to any one of claims 1 to 5, characterised in that the solvent medium is anhydrous methanol used in a minimum quantity for the solubilization of the substrate.

7. Process for the production of aromatic aldehydes according to any one of claims 1 to 6, characterised in that the oxidation reaction is carried out at a temperature of between 55 and 65 °C, preferably at 60 °C.

8. Process for the production of aromatic aldehydes according to any one of claims 1 to 7, characterised in that the base constituted by sodium hydroxide is used in a NaOH/substrate ratio of between 1 and 6, preferably between 2.5 and 4.5.

9. Process for the production of aromatic aldehydes according to any one of claims 1 to 8, characterised in that the alkyl phenol is methyl-4 phenol, dimethyl-2,4-phenol, trimethyl-2,4,6 phenol, ditertiobutyl-2,6 methyl-4 phenol or bromo-2-methyl-4 phenol.

10. Process for the production of aromatic aldehydes represented by the formula

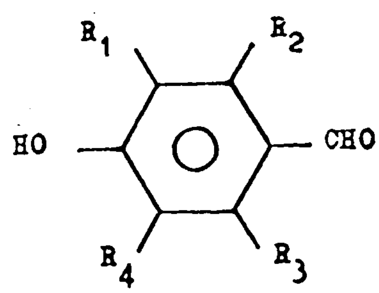

in which $R_1$, $R_2$, $R_3$, $R_4$ are together or separately hydrogen or a lower alkyl radical or a halogen atom, by the reaction of an alkyl phenol of the formula

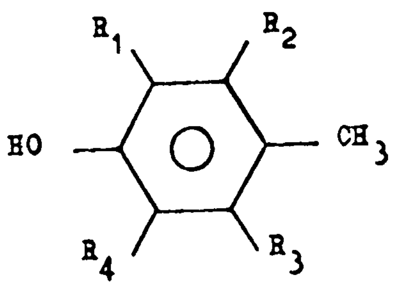

in which $R_1$, $R_2$, $R_3$ and $R_4$ have the same meaning as before, with oxygen, in a solvent medium, in the presence of a base and a catalytic quantity of a cobalt compound, characterised in that the substrate oxidation reaction is carried out under the following conditions:

a) in the presence of a catalyst constituted by a poorly oxidizable, chelate complex of cobalt (II) having a rigid structure, chosen from the group comprising bis-(4-methylpyridine isoindolinato) cobalt (II) acetate, phthalocyaninato-cobalt (II) and sulphophthalocyaninato-cobalt (II);
b) the catalyst is used in a quantity of between 1‰ and 5% relative to the substrate;
c) the oxidizing gas is constituted by pure oxygen or oxygen-nitrogen mixtures with a high oxygen content;
d) oxidation is carried out under normal pressure and at a temperature of between 55 and 65 °C;
e) the base constituted by sodium hydroxide is used in a NaOH/substrate ratio of between 1 and 6.

## Patentansprüche

1. Verfahren zur Herstellung aromatischer Aldehyde der Formel

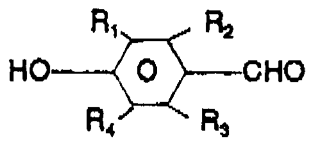

worin $R_1$, $R_2$, $R_3$ und $R_4$ zugleich oder getrennt voneinander Wasserstoff, einen niedermolekularen Alkylrest oder Halogen bedeuten, durch Umsetzung eines Alkylphenols der Formel

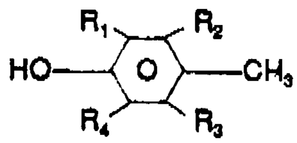

worin $R_1$, $R_2$, $R_3$ und $R_4$ die gleichen Bedeutungen wie oben haben, mit Sauerstoff in einem Lösungsmittelmedium in Gegenwart einer Base und einer katalytischen Menge einer Kobaltverbindung, **dadurch gekennzeichnet,** daß die Oxidation des Substrates in Gegenwart eines Katalysators durchgeführt wird, der aus einem Kobalt(II)-chelatkomplex von wenig oxidierbarer starrer Struktur besteht und aus der Gruppe Bis-(4-methylpyridinisoindolinato)-Kobalt(II)-acetat, Phthalocyaninato-Kobalt(II) und Sulfophthalocyaninato-Kobalt(II) ausgewählt ist.

2. Verfahren zur Hertellung aromatischer Aldehyde nach Anspruch 1, **dadurch gekennzeichnet,** daß das Phthalocyaninato-Kobalt(II) und das Sulfophthalocyaninato-Kobalt(II) auf einem Träger adsorbiert sind.

3. Verfahren zur Herstellung aromatischer Aldehyde nach Anspruch 1, **dadurch gekennzeichnet,** daß die Oxidation in Gegenwart von 1 ‰ bis 5 % Katalysator bezogen auf das Alkylphenol durchgeführt wird.

4. Verfahren zur Herstellung aromatischer Aldehyde nach einem der Ansprüche 1 bis 3, **dadurch gekennzeichnet,** daß das oxidierende Gas aus reinem Sauerstoff oder Sauerstoff-Stickstoffgemischen mit erhöhtern Sauerstoffgehalt zwischen 40 und 90 Vol.-% besteht.

5. Verfahren zur Hertellung aromatischer Aldehyde nach einem der Ansprüche bis 4, **dadurch gekennzeichnet,** daß die Oxidation unter Normaldruck von etwa 0,1 MPa durchgeführt wird.

6. Verfahren zur Herstellung aromatischer Aldehyde nach einem der Ansprüche 1 bis 5, **dadurch gekennzeichnet,** daß das Lösemittelmedium wasserfreies Methanol ist, welches in der für die Löslichmachung des Substrates kleinsten Menge benutz wird.

7. Verfahren zur Herstellung aromatischer Aldehyde nach einem der Ansprüche bis 6, **dadurch gekennzeichnet,** daß die Oxidation bei einer Temperatur zwischen 55 und 65 °C, vorzugsweise bei 60 °C, durchgeführt wird.

8. Verfahren zur Herstellung aromatischer Aldehyde nach einem der Ansprüche 1 bis 7, **dadurch gekennzeichnet,** daß die Base aus Natriumhydroxid besteht und in einem Verhältnis von NaOH/Substrat zwischen 1 und 6, vorzugsweise zwischen 2,5 und 4,5, verwendet wird.

9. Verfahren zur Herstellung aromatischer Aldehyde nach einem der Ansprüche 1 bis 8, **dadurch gekennzeichnet,** daß das Alkylphenol 4-Methylphenol, 2,4-Dimethylphenol, 2,4,6-Trimethylphenol, 2,6-Ditertiärbutyl-4-methylphenol oder 2-Brom-4-methylphenol ist.

10. Verfahren zur Herstellung aromatischer Aldehyde der Formel

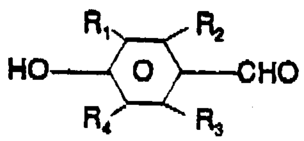

worin $R_1$, $R_2$, $R_3$ und $R_4$ zugleich oder getrennt voneinander Wasserstoff, einen niedermolekularen Alkylrest oder Halogen bedeuten, durch Umsetzung eines Alkylphenol der Formel

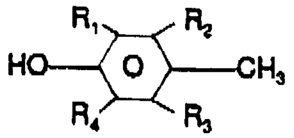

worin $R_1$, $R_2$, $R_3$ und $R_4$ die gleichen Bedeutungen wie oben haben, mit Sauerstoff in einem Lösungsmittelmedium in Gegenwart einer Base und einer katalytischen Menge einer Kobaltverbindung, **dadurch gekennzeichnet,** daß die Oxidation des Substrates unter den folgenden Bedingungen durchgeführt wird:

a) in Gegenwart eines Katalysators, der aus einem Kobalt(II)-chelatkomplex wenig oxidierbarer starrer Struktur besteht, der aus der Gruppe Bis-(4-methylpyridinisoindolinato)-Kobalt(II)-acetat, Phthalocyaninato-Kobalt(II) und Sulfophthalocyaninato-Kobalt(II) ausgewählt ist;

b) der Katalysator wird in einer Menge zwischen 1 ‰ und 5 %, bezogen auf das Substrat, vewendet;

c) das oxidierende Gas besteht aus reinem Sauerstoff oder Sauerstoff-Stickstoffgemischen mit erhöhtem Sauerstoffgehalt;

d) die Oxidation wird unter Normaldruck und bei einer Temperatur zwischen 55 und 65 °C durchgeführt;

e) die aus Natriumhydroxid bestehende Base wird in einem Verhältnis NaOH/Substrat zwischen 1 und 6 verwendet.